Europäisches Patentamt

European Patent Office

Office européen des brevets

(19)

(11) Veröffentlichungsnummer : **0 519 900 A1**

# (12) EUROPÄISCHE PATENTANMELDUNG

(21) Anmeldenummer : **92890144.6**

(22) Anmeldetag : **16.06.92**

(51) Int. Cl.$^5$ : **A61K 37/547**

(30) Priorität : **20.06.91 AT 1240/91**

(43) Veröffentlichungstag der Anmeldung :
**23.12.92 Patentblatt 92/52**

(84) Benannte Vertragsstaaten :
**AT BE CH DE DK ES FR GB IT LI NL SE**

(71) Anmelder : **IMMUNO Aktiengesellschaft
Industriestrasse 67
A-1221 Wien (AT)**

(72) Erfinder : **Eibl, Johann, Dr.
Gustav Tschermakgasse 2
A-1180 Wien (AT)**
Erfinder : **Philapitsch, Anton
Hans Kudlichgasse 5
A-2490 Ebenfurt (AT)**
Erfinder : **Schwarz, Hans Peter, Doz. Dr.
Schindlergasse 32
A-1180 Wien (AT)**

(74) Vertreter : **Wolfram, Gustav, Dipl.-Ing. et al
Patentanwälte Sonn, Pawloy, Weinzinger &
Wolfram, Riemergasse 14
A-1010 Wien (AT)**

(54) **Pharmazeutische Präparation enthaltend eine thrombolytisch wirkende Substanz und Protein C.**

(57)    Die Erfindung betrifft eine pharmazeutische Präparation enthaltend eine thrombolytisch wirkende, Protein C nicht aktivierende Substanz und Protein C. Diese Präparation verhindert eine Reocclusion, welche üblicherweise im Rahmen einer Thrombolysetherapie auftritt.

EP 0 519 900 A1

Jouve, 18, rue Saint-Denis, 75001 PARIS

Die Erfindung betrifft eine pharmazeutische Präparation enthaltend Protein C.

Viele operative Eingriffe erhöhen das Risiko einer venösen und arteriellen Thrombose und Thromboembolie. Arterielle und venöse Thrombosis kann auch krankheitsbedingt sein. Die antithrombotische bzw. thrombolytische Therapie bringt jedoch unerwünschte Nebeneffekte, wie Blutungen oder Reocclusion mit sich.

Die thrombolytische Wirkung von Substanzen, wie t-PA, Urokinase, Streptokinase oder Plasminogen beruht auf der Freisetzung von Plasmin, welches Plasmin die Auflösung von Thromben ermöglicht. Gleichzeitig wird beobachtet, daß bei Verabreichung thrombolytisch wirksamer Substanzen Thrombin generiert wird. Es wird vermutet, daß die Thrombin-Generierung und eine darauffolgende Reocclusion ein allgemeiner unerwünschter Nebeneffekt einer erfolgreichen Thrombolyse ist (Circulation 83, 937-944, (1991)).

Neben der erhöhten Thrombinaktivität wird auch eine Veränderung der Protein C-Konzentration im Blut von Thrombolyse-Patienten beobachtet (Seminars in Thrombosis and Hemostasis 16, 242-244 (1990)). Die Therapie erfolgt entweder mit t-PA oder mit Heparin oder mit einer Kombination aus t-PA und Heparin. Eine Verringerung der Protein C-Konzentration wird nur bei Verabreichung der t-PA-hältigen Präparate beobachtet. Ein direkter Abbau von Protein C mit t-PA oder Plasmin wird jedoch ausgeschlossen.

Im Zusammenhang mit unerwünschten Nebenwirkungen der Thrombolyse wird in der EP-A - 0 318 201 vorgeschlagen, aktiviertes Protein C (aPC) alleine oder in Kombination mit einem Thrombolytikum zur Verhinderung akuter arterieller thrombotischer Verschlüsse, Thromboembolie oder Stenosis zu verwenden. aPC gilt als antikoagulativ wirksames Enzym, welches einerseits eine Thrombinbildung durch den proteolytischen Abbau von aktiviertem Gerinnungsfaktor V und aktiviertem Gerinnungsfaktor VIII unterbindet und andererseits die Fibrinolyse unterstützt. Aus diesem Grund kann die Dosis des Thrombolytikums reduziert werden.

Gleichermaßen wird in Blood 74, Suppl. 1, 50a, Abstract 176 (1989) zur Verhinderung der Thrombenbildung ein Kombinationspräparat enthaltend aPC und Urokinase vorgeschlagen, um die thrombolytische oder antithrombotische Dosis der Urokinase zu verringern und gleichzeitig den Blutungskomplikationen, welche bei einer Thrombolysetherapie auftreten, zu begegnen. Die Verabreichung von aktiviertem Protein C hat jedoch den Nachteil, daß aufgrund der unmittelbar antikoagulatorischen Wirksamkeit von aPC die Blutungsneigung gefördert wird.

Die Erfindung stellt sich die Aufgabe, diesen Nachteil zu beseitigen und eine pharmazeutische Präparation zur Verfügung zu stellen, welche eine Reocclusion, bedingt durch eine erhöhte Thrombinaktivität nach erfolgter Thrombolysetherapie, verhindert.

Die erfindungsgemäße Präparation enthält eine thrombolytisch wirkende, Protein C nicht aktivierende Substanz und Protein C.

Es hat sich gezeigt, daß die erfindungsgemäße Präparation eine erfolgreiche Thrombolysetherapie ohne Reocclusionsgefahr ermöglicht.

Als thrombolytisch wirkende Substanz eignet sich besonders Urokinase, tPA, Lys-Plasminogen oder Streptokinase.

Die Erfindung betrifft daher auch die Verwendung einer thrombolytisch wirkenden Substanz, welche Protein C nicht zu aktivieren vermag, in Kombination mit Protein C zur Herstellung eines Arzneimittels zur Behandlung von Thrombosen und zur Verhinderung der Reocclusion.

Die Erfindung beruht auf der Erkenntnis, daß das durch therapeutische Thrombolytika generierte Plasmin nicht nur Fibrin abbaut, sondern bei den während einer Thrombolysetherapie sehr hohen Konzentrationen überraschenderweise auch Protein C inaktiviert, was einen Protein C-Mangel hervorruft und einen hyperkoagulabilen Zustand auslöst. Das kann verhindert werden, wenn die thrombolytisch wirkende Substanz in Kombination mit Protein C verabreicht wird oder wenn Protein C im Rahmen einer Thrombolysetherapie, also vor, während und/oder nach einer Thrombolysetherapie verabreicht wird.

Im erfindungsgemäßen Präparat ist Protein C vorzugsweise zu 10 bis 50 E/mg Protein enthalten. Die applikationsbereite Form sollte 90 bis 450 E/ml enthalten. Der Gehalt an Thrombolytikum wird in der erfindungsgemäßen Präparation zweckmäßigerweise so gewählt, daß bei der Applikation übliche Mengen verabreicht werden.

Die erfindungsgmeäße Protein C-hältige Präparation kann als Injektion (30 bis 80 E/kg) 2 oder 3 mal täglich oder als Dauerinfusion (15 bis 30 E/kg.h) verabreicht werden. Nachfolgend wird die Erfindung noch näher beschrieben.

Gewinnung von Protein C

Hochreines Protein C wurde aus einer rohen Protein C-Fraktion gewonnen, die aus kommerziell erhältlichem Prothrombinkomplex-Konzentrat hergestellt wurde. Die Reinigung erfolgte affinitätschromatographisch mittels monoklonaler Antikörper. Monoklonale Anti-Protein C-Antikörper wurden wie folgt hergestellt:

BALB/C-Mäuse wurden mit 100 μg menschlichem Protein C in zweiwöchigen Abständen durch intraperitoneale Injektion immunisiert. Nach sechs Wochen wurden noch einmal 50 μg des menschlichen Protein C injiziert und drei Tage später die Fusion vorgenommen. Die Myelomzellinie (P3-X-63-AG8-653, $1,5 \times 10^7$ Zellen) wurde vermischt mit $1,7 \times 10^8$ Milz-

zellen von einer Maus, und die Fusion nach modifizierter Köhler & Milestein-Methode unter Verwendung von PEG 1500 durchgeführt (Köhler G., Milestein C., Nature 256(1975)495-497).

Positive Klone, getestet mittels eines ELISA, wurden zweimal subgeklont. Aszitesproduktion erfolgte durch Injektion von $5 \times 10^6$ Hybridomzellen je BALB/C-Maus zwei Wochen nach Pristan-Behandlung.

Das Immunglobulin wurde aus Aszites durch Ammoniumsulfatpräzipitation und anschließende Chromatographie mittels QAE-Sephadex und darauffolgend Chromatographie an Sephadex G200 gereinigt. Um das Risiko der Übertragung muriner Viren zu reduzieren, wurde der Antikörper vor der Immobilisierung noch einem Virusinaktivierungsschritt unterworfen. Die so erhaltenen monoklonalen Antikörper gegen Protein C wurden an CNBr-aktivierte Sepharose 4B (Pharmacia) gekoppelt. Für die Reinigung des Protein C mittels Affinitätschromatographie wurden folgende Puffer verwendet:

Als Absorptionspuffer: 20 mmol Tris, 2 mmol EDTA, 0,25 mol NaCl und 5 mmol Banzamidin;
als Waschpuffer wurde verwendet: 20 mmol Tris, 1 mol NaCl, 2 mmol Benzamidin, 2 mmol EDTA; der pH-Wert betrug 7,4;
als Elutionspuffer wurde verwendet: 3 mol NaSCN, 20 mmol Tris, 1 mol NaCl, 0,5 mmol Benzamidin, 2 mmol EDTA.

Im einzelnen: Das Prothrombinkomplex-Konzentrat wurde im Absorptionspuffer aufgelöst, wobei etwa 10 g des Prothrombinkomplex-Konzentrates für eine 20 ml monoklonale Antikörpersäule zur Verwendung kamen. Anschließend wurde das aufgelöste Prothrombinkomplex-Konzentrat filtriert, bei 20.000 rpm 15 min lang zentrifugiert und durch ein Konzentrat filtriert, bei 20.000 rpm 15 min lang zentrifugiert und durch ein 0,8 μm Filter sterilfiltriert. Das sterilfiltrierte und gelöste Prothrombinkomplex-Konzentrat wurde auf die Säule aufgetragen mit einer Flußrate von 10 ml/h. Anschließend wurde die Säule mit dem Waschpuffer proteinfrei gewaschen und schließlich das gebundene Protein C mit dem Elutionspuffer bei einer Flußrate von 5 ml/h eluiert und die Fraktionen gesammelt. Das eluierte Protein C wurde gegen einen Puffer (0,2 mol/l Tris, 0,15 mol Glycin und 1 mmol EDTA, pH 8,3) dialysiert. Der Protein C-Gehalt wurde antigenmäßig mittels der Methode nach Laurell und aktivitätsmäßig nach Protac-Aktivierung bestimmt.

Das erhaltene Protein C-Eluat wurde in folgender Weise zu einer pharmazeutisch applizierbaren Präparation fertiggestellt:

Das Eluat wurde zuerst einem Ultrafiltrations- und einem Diafiltrationsschritt unterworfen. Für die Diafiltration wurde ein Puffer mit einem pH-Wert von 7,4 verwendet, der pro Liter 150 mMol NaCl und 15 mMol Trinatriumcitrat.$2H_2O$ enthielt. Das erhaltene Filtrat wurde gefriergetrocknet und durch eine einstündige Dampfbehandlung bei 80° C $\pm$ 5° C und 1375 $\pm$ 35 mbar virusinaktiviert.

Das lyophilisierte virusinaktivierte Material wurde sodann in einer sterilen isotonen NaCl-Lösung gelöst und mittels Ionenaustauschchromatographie an Q-Sepharose wurden etwaig vorhandene Antikörper bzw. Serumamyloid P entfernt. Die gereinigte Lösung wurde durch einen weiteren Ultrafiltrations- und Diafiltrationsschritt konzentriert. Danach wurden zur erhaltenen Lösung pro Liter 10 g Albumin, 150 mMol NaCl und 15 mMol Trinatriumcitrat zugegeben. Der pH-Wert der Lösung betrug 7,5. Maus-Immunglobulin sowie die Faktoren II, VII, IX und X konnten nicht nachgewiesen werden. Anschließend wurde die Lösung sterilfiltriert, abgefüllt und lyophilisiert. Die spezifische Aktivität betrug 14 Einheiten Protein C/mg. Eine Einheit Protein C Aktivität ist definiert als die Protein-C-Aktivität in 1 ml Normalplasma und wird gegen den ersten internationalen Standard von Protein C kalibriert. Als Aktivitätstest wurde ein amidolytischer Test verwendet, wobei das Protein C mittels Protac (Fa. Pentapharm), einem gängigen Protein C-Aktivator, hergestellt aus einem Schlangengiftpräparat, aktiviert wurde.

Zeitabhängiger Abbau von Protein C

Protein C wurde mit Plasmin behandelt und der Abbau mittels Immunoblot verfolgt. Dazu wurden 270 μl einer Protein C-hältigen Lösung (8 μg/ml) mit 270 μl Plasmin (1 CU/ml) bei 37°C inkubiert. Das Substrat/Enzym-Verhältnis betrug demnach 8 : 1 (μg/CU). Nach bereits 60 min ließ sich Protein C amidolytisch nicht mehr nachweisen.

Dosisabhängiger Abbau von Protein C

Um den dosisabhängigen Abbau von plasmatischem Protein C zu untersuchen, wurden zu je 50 μl humanem Citratplasma je 50 μl Plasmin in Konzentrationen von 10, 5, 3, 1,5 und 0,5 CU/ml zugesetzt. Nach einer Reaktionszeit von 10 min wurden je 50 μl Antithrombin III-Heparin-Komplex (10 E ATIII, 50 E Heparin pro ml) zugefügt. Dieser Zusatz stoppt die Reaktion.

Protein C wurde nach Aktivierung mit Protac (Firma Pentapharm) amidolytisch mit dem spezifischen chromogenen Substrat S 2366 (Fa. Kabi) bestimmt.

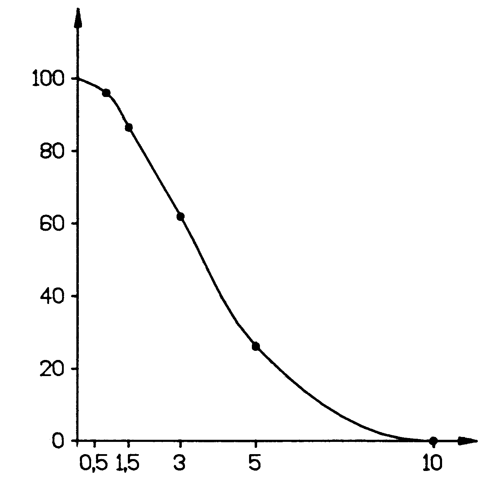

Als Vergleich wurde plasmatisches Protein C ohne Plasminzusatz parallel dazu behandelt. Die Ergebnisse sind aus der Abbildung (Abszisse: CU Plasmin; Ordinate: % Protein C-Aktivität) ersichtlich. Die Abbildung zeigt, daß bei einem Zusatz einer Lösung mit 10 CU/ml Plasmin Protein C bereits nach 10 min vollständig abgebaut wurde.

**Patentansprüche**

1. Pharmazeutische Präparation enthaltend eine thrombolytisch wirkende, Protein C nicht aktivierende Substanz und Protein C.

2. Pharmazeutische Präparation nach Anspruch 1, dadurch gekennzeichnet, daß als thrombolytisch wirkende Substanz Urokinase, tPA, Lys-Plasminogen oder Streptokinase vorgesehen ist.

3. Verwendung einer thrombolytisch wirkenden Substanz, welche Protein C nicht zu aktivieren vermag, in Kombination mit Protein C zur Herstellung eines Arzneimittels zur Behandlung von Thrombosen und zur Verhinderung der Reocclusion.

4. Verwendung von Protein C zur Herstellung eines Arzneimittels zur Verhinderung der Reocclusion im Rahmen einer Thrombolysetherapie.

Europäisches
Patentamt

**EUROPÄISCHER RECHERCHENBERICHT**

Nummer der Anmeldung

EP    92 89 0144

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 215 548 (ZYMOGENETICS INC) 25. März 1987 <br> * Anspruch 19 * <br> --- | 4 | A61K37/547 |
| X | EP-A-0 326 014 (HOECHST JAPAN LTD) 2. August 1989 <br> * Zusammenfassung; Ansprüche * <br> --- | 4 | |
| A | EP-A-0 318 201 (SCRIPPS CLINIC AND RESEARCH FOUNDATION) 31. Mai 1989 <br> * das ganze Dokument * <br><br> ----- | 1-3 | |
| | | | **RECHERCHIERTE SACHGEBIETE (Int. Cl.5)** <br><br> C12N <br> A61K |

Der vorliegende Recherchenbericht wurde für alle Patentansprüche erstellt

| Recherchenort | Abschlußdatum der Recherche | Prüfer |
|---|---|---|
| DEN HAAG | 26 AUGUST 1992 | VAN DER SCHAAL C.A. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.............................................................................
& : Mitglied der gleichen Patentfamilie, übereinstimmendes
    Dokument

EPO FORM 1503 03.82 (P0403)